# EUROPÄISCHE PATENTSCHRIFT

(11) **EP 2 500 335 B1**
(45) Veröffentlichungstag und Bekanntmachung des Hinweises auf die Patenterteilung: **09.09.2015**
(21) Anmeldenummer: 12159356.0
(22) Anmeldetag: 14.03.2012
(51) Int. Cl.: C07C 41/58, C07C 43/04

(54) **Verfahren zur Abtrennung von Ethylal aus Ethanol und Ethylal enthaltenden Gemischen**
Method for separating ethylal from ethanol and mixtures containing ethylal
Procédé de séparation d'éthylal à partir de mélanges contenant de l'éthanol et de l'éthylal

(30) Priorität: 18.03.2011 EP 11158894
(43) Veröffentlichungstag der Anmeldung: 19.09.2012
(73) Patentinhaber: Technische Universität Kaiserslautern, 67663 Kaiserslautern (DE)
(72) Erfinder: Ströfer, Eckhard, 68163 Mannheim (DE); Hasse, Hans, 67661 Kaiserslautern (DE); Burger, Jakob, 67663 Kaiserslautern (DE); Drunsel, Jan-Oliver, 63477 Maintal (DE)
(74) Vertreter: Schuck, Alexander

(56) Entgegenhaltungen:
- WO-A1-88/07989
- WO-A1-94/17024

## Beschreibung

### Beschreibung

Die Erfindung betrifft ein Verfahren zur Abtrennung von Ethylal aus einem Gemisch, das Ethanol, Ethylal und gegebenenfalls Wasser enthält, insbesondere aus einem Gemisch, das bei der Synthese von Ethylal aus Ethanol und Formaldehyd anfällt.

Ethylal kann als die Rußbildung inhibierendes Kraftstoffadditiv verwenden werden, daher besteht eine steigende Nachfrage nach Ethylal. Neben Ethylal kommen auch Ethylal-Folgeprodukte, wie Poly(oxymethylen)diethylether, als Kraftstoffadditive in Betracht. Die vorliegende Erfindung kann großtechnisch in kontinuierlichen Ethylal-Prozessen zum Einsatz kommen.

Ethylal entsteht durch sauer katalysierte Reaktion von Formaldehyd mit Ethanol nach folgender Summengleichung:
2 Ethanol + Formaldehyd <--> Ethylal + Wasser

Bei der Synthese fallen Gemische an, die Ethanol, Ethylal und Wasser und gegebenenfalls noch weitere Stoffe wie z.B. Formaldehyd und Methylformiat enthalten.

Das Verfahren zur Führung und Katalyse der Reaktion ist in DD245868 beschrieben. Die Umsetzung ist weiterhin beschrieben in S. Chopade, M. Sharma, Reaction of ethanol and formaldehyde: use of versatile cation-exchange resins as catalyst in batch reactors and reactive distillation columns; Reactive & Functional Polymers, 32 (1997), 53-64.

Die Reaktion ist reversibel und nicht vollständig, sodass nach der Reaktion im Allgemeinen Mischungen von Ethylal, Ethanol, Wasser und auch Formaldehyd vorliegen. Formaldehyd liegt in diesen Gemischen vorwiegend in Form von Methylenglykol und Polyoxymethylenglykolen sowie in Form von deren Hemiacetalen mit Ethanol vor.

Durch das Vorhandensein eines Azeotrops im Binärsystem Ethylal-Ethanol gelingt die Gewinnung von reinem Ethylal aus binären Ethylal-Ethanol-Gemischen durch einfache Rektifikation nicht. Weiterhin tritt im Ternärsystem Ethylal-Ethanol-Wasser ein ternäres Leichtsiederazeotrop auf, das die Auftrennung von Wasser enthaltenden Ethylal-Ethanol-Gemischen zusätzlich erschwert. Für die Abtrennung von Ethylal aus diesen Gemischen sind verschiedene physikalische oder chemische Verfahren bekannt.

In US 4,613,411 wird vorgeschlagen, aus dem Reaktionsgemisch der Ethylal-Herstellung in einer ersten Rektifikationsstufe ein azeotropes Gemisch aus Ethylal und Ethanol abzutrennen. Dem Gemisch aus Ethylal und Ethanol wird ein Hilfsstoff, z.B. Cyclohexan, beigemischt, mit dem Ethanol ein Leichtsiederazeotrop bildet, und dieses Azeotrop in einer zweiten Rektifikationsstufe von Ethylal abgetrennt.

CN 1388107 A, CN 1537839 A, CN 1721024 A und CN 101503342 A offenbaren Verfahren der Extraktion von Ethylal aus einem azeotropen Gemisch Ethylal-Ethanol-Gemisch, welches in einer ersten Rektifikationsstufe anfällt. Als Extraktionsmittel für Ethylal werden vorzugsweise Polyole - ausdrücklich genannt wird Glycerin - eingesetzt.

Gemäß WO 94/17024 wird die Umsetzung von Formaldehyd mit Ethanol in Methylenchlorid als Lösungsmittel durchgeführt. Dabei wird während der Reaktion gebildetes Wasser kontinuierlich mittels eines Wasserabscheiders abgeschieden, wodurch sehr hohe Umsätze erzielt werden können. Ethanol reagiert dabei fast vollständig mit Formaldehyd zu Ethylal ab. Ethylal und Methylenchlorid werden anschließend durch einfache Destillation aufgetrennt.

Gemäß WO 88/07989 wird eine Mischungslücke im System Ethylal-Ethanol-Wasser ausgenutzt. Zunächst wird in einer ersten Rektifikationsstufe das ternäre Azeotrop gewonnen. Nach Zugabe von Wasser zerfällt dieses in eine Ethylal-arme und eine Ethylal-reiche Phase. Aus der Ethylal-reichen Phase wird in einer weiteren Rektifikationsstufe reines Ethylal als Sumpfabzug gewonnen.

Alle beschriebenen Prozesse setzen Hilfsstoffe ein. Eine einfache Entsorgung der Hilfsstoffe ohne vorherige Aufarbeitung stellt unter Umweltschutzaspekten keine Option dar. Zudem würden gelöste Wertprodukte wie Ethanol dabei verloren gehen. Daher müssen die Hilfsstoffe unter großem Aufwand aufgearbeitet werden. Der Prozess gemäß WO88/07989 lässt die Gewinnung von reinem Ethylal ohne Einsatz von Hilfsstoffen zu, jedoch muss Wasser im Zulauf vorhanden sein oder zugegeben werden. Der Prozess ist durch den Einsatz mehrerer Rektifikationsstufen sowie einer Flüssig-Flüssig-Extraktionsstufe apparativ sehr aufwendig. Durch die ungünstige Lage des ternären Azeotrops und der Mischungslücke sind hohe Rückführströme erforderlich, die das Verfahren energieaufwändig machen.

Aufgabe der Erfindung ist es, ein einfaches Verfahren zur Abtrennung von Ethylal aus Ethylal und Ethanol enthaltenden Gemischen, insbesondere aus Ethylal, Ethanol und Wasser enthaltenden Gemischen, wie sie bei der Herstellung von Ethylal aus Formaldehyd und Wasser anfallen, bereitzustellen. Das Verfahren soll kostengünstig, energieeffizient und umweltverträglich sein und ohne den Einsatz von Hilfsstöffen und zusätzlichem Wasser auskommen.

Gelöst wird die Aufgabe durch ein Verfahren zur Abtrennung von Ethylal aus einem Einsatzstrom 1, der Ethanol, Ethylal und gegebenenfalls Wasser enthält, bei dem
a) ein Einsatzstrom 1, der Ethanol und Ethylal enthält, bereitgestellt wird,
b) der Einsatzstrom 1 und ein Rückführstrom 5, der Ethanol und Ethylal enthält, in eine erste Destillationsstufe eingespeist und bei einem Druck von 0,1 bis 32 bar destilliert werden, wobei ein Strom 2, der Ethanol und Ethylal enthält, wobei dieser Strom Ethylal in höherer Konzentration als Strom 1 enthält, erhalten wird,
c) der Strom 2 in einer zweiten Destillationsstufe bei einem Druck von 1,1 bis 33 bar destilliert wird, wobei der Druck in der zweiten Destillationsstufe um 1,0 bis 10 bar höher als der Druck in der ersten Destillationsstufe ist, wobei ein Rückführstrom 5, der Ethanol und Ethylal enthält, und ein Strom 6, der im Wesentlichen aus Ethylal besteht, erhalten werden.

"Im Wesentlichen bestehend aus" im Sinne der vorliegenden Erfindung heißt, dass die betreffende Komponente bzw. die betreffenden Komponenten mindestens 90 Gew.-%, bevorzugt mindestens 95 Gew.-% und insbesondere mindestens 98 Gew.-% des Gemischs ausmachen.

Vorzugsweise ist der Druck in der zweiten Destillationsstufe um 3 bis 6 bar höher als der Druck in der ersten Destillationsstufe.

Vorzugsweise wird die erste Destillationsstufe bei einem Druck von 0,25 bis 1,5 bar, besonders bevorzugt bei einem Druck von ca. 1 bar (0,9 bis 1,1 bar) durchgeführt.

Im Allgemeinen werden die erste und die zweite Destillationsstufe in Destillationskolonnen durchgeführt. Die erste Destillationsstufe wird im Allgemeinen in einer ersten Destillationskolonne mit mindestens 2 theoretischen Böden, vorzugsweise 2 bis 50, besonders bevorzugt 4 bis 25 theoretischen Stufen durchgeführt. Die zweite Destillationsstufe wird im Allgemeinen in einer zweiten Destillationskolonne mit mindestens 2 theoretischen Böden, vorzugsweise 2 bis 50, besonders bevorzugt 4 bis 25 theoretischen Stufen durchgeführt.

Vorzugsweise wird der Strom 2 als Kopfabzugsstrom und der Strom 6 als Sumpfabzugsstrom erhalten.

Der Einsatzstrom 1 enthält Ethanol als Hauptkomponente, d.h. die Komponente mit dem größten Gewichtsanteil.

In einer Ausführungsform der Erfindung besteht der Einsatzstrom 1 im Wesentlichen aus Ethanol und Ethylal. Dabei wird in Schritt b) ein Strom 3, der im Wesentlichen aus Ethanol besteht, als Sumpfabzugsstrom erhalten.

Im Allgemeinen enthält der Einsatzstrom 1 Wasser. In einer Ausführungsform des erfindungsgemäßen Verfahrens wird in Schritt b) ein Strom 3, der im Wesentlichen aus Ethanol und Wasser besteht, als Sumpfabzugsstrom erhalten.

In einer weiteren Ausführungsform enthält der Einsatzstrom 1 Wasser, und in Schritt b) werden ein Strom 4, der Ethanol und Wasser enthält, als Seitenabzugsstrom und ein Strom 3, der im Wesentlichen aus Wasser besteht, als Sumpfabzugsstrom erhalten.

Der Einsatzstrom 1 kann Leichtsieder enthalten. Leichtsieder sind beispielsweise Methylformiat, Ethylformiat und Dimethylether.

In einer weiteren Ausführungsform der Erfindung enthält der Einsatzstrom 1 Leichtsieder, und wird in Schritt c) werden der Rückführstrom 5 als Seitenabzugsstrom und ein Strom 7, der Leichtsieder enthält, als Kopfabzugsstrom erhalten.

Der Einsatzstrom 1 kann darüber hinaus Formaldehyd enthalten.

In einer weiteren Ausführungsform der Erfindung enthält der Einsatzstrom 1 Formaldehyd und Wasser, und in Schritt b) ein Strom 3, der im Wesentlichen aus Ethanol, Wasser und Formaldehyd besteht, als Sumpfabzugsstrom erhalten.

In einer weiteren Ausführungsform der Erfindung enthält der Einsatzstrom 1 Formaldehyd und Wasser, und in Schritt b) werden ein Strom 4, der Ethanol und Wasser enthält, als Seitenabzugsstrom und ein Strom 3, der im Wesentlichen aus Wasser und Formaldehyd besteht, als Sumpfabzugsstrom erhalten.

Typischer Weise enthält der Einsatzstrom 1 20 bis 85 Gew.-% Ethanol, 5 bis 40 Gew.-% Ethylal und 10 bis 40 Gew.-% Wasser. Der Strom 3, der als Sumpfabzugsstrom der ersten Destillationskolonne erhalten wird, enthält im Allgemeinen weniger als 1 Gew.-%, bevorzugt weniger als 0,1 Gew.-% Ethylal. Beispielsweise setzt sich der Strom 3 wie folgt zusammen: 33 bis 89 Gew.-% Ethanol, 11 bis 67 Gew.-% Wasser und 0 bis 0,05 Gew.-% Ethylal.

Der Strom 2, der im Allgemeinen als Kopfabzugsstrom der ersten Destillationskolonne erhalten wird, enthält im Allgemeinen 40 bis 60 Gew.-%, vorzugsweise 45 bis 55 Gew.-% Ethylal. Der Strom 2 kann bis zu 10 Gew.-%, vorzugsweise bis zu 2 Gew.-% Leichtsieder enthalten. Beispielsweise setzt sich der Strom 2 wie folgt zusammen: 28 bis 52 Gew.-% Ethanol, 45 bis 55 Gew.-% Ethylal, 3 bis 15 Gew.-% Wasser und 0 bis 2 Gew.-% Leichtsieder. Der Rückführstrom 5, der als Kopfabzugsstrom oder Seitenabzugsstrom der zweiten Destillationskolonne erhalten wird, enthält beispielsweise 35 bis 57 Gew.-% Ethanol, 40 bis 55 Gew.-% Ethylal und 3 bis 15 Gew.-% Wasser. Enthält der Strom 2 Leichtsieder, können diese als Kopfabzugsstrom der zweiten Destillationskolonne abgetrennt werden. Der Sumpfabzugsstrom 6 der zweiten Kolonne enthält im Allgemeinen mindestens 99 Gew.-%, bevorzugt mindestens 99,9 Gew.-% Ethylal.

In einer Variante wird ein Seitenabzugsstrom 4 im Abtriebsteil der ersten Destillationskolonne vorgesehen. Im Allgemeinen umfasst der Abtriebsteil dieser Destillationskolonne 25 bis 90 %, vorzugsweise 50 bis 75 % der theoretischen Stufen dieser Kolonne. Beispielsweise enthält dieser Seitenabzugsstrom 4 90 bis 98 Gew.-% Ethanol und 2 bis 10 Gew.-% Wasser. Der Sumpfabzugsstrom 3 enthält dann im Allgemeinen mindestens 95 Gew.-% Wasser, bevorzugt mindestens 99,9 Gew.-% Wasser.

Der Einspeisungsstrom 1 kann weiterhin Formaldehyd enthalten. Der Einspeisungsstrom 1 kann im Allgemeinen bis zu 10 Gew.-% Formaldehyd, beispielsweise 0,5 bis 2 Gew.-% Formaldehyd enthalten. Formaldehyd wird im Allgemeinen zu über 80%, vorzugsweise zu über 90% im Sumpfabzugsstrom 3 der ersten Kolonne ausgetragen. In allen anderen Abzugsströmen beträgt der Formaldehydanteil weniger als 1 Gew-%, vorzugsweise weniger als 0,1 Gew-%.

In einer bevorzugten Ausführungsform enthält die erste Rektifikationskolonne eine Reaktionszone, in der ein saurer heterogener Katalysator angeordnet ist. In dieser Reaktionszone reagieren Formaldehyd und Ethanol zu weiterem Ethylal. Im Allgemeinen ist diese Reaktionszone im Abtriebsteil der ersten Kolonne angeordnet.

Geeignete saure Katalysatoren sind insbesondere saure lonentauscherharze, Zeolithe, Aluminosilikate, Siliziumdioxid, Aluminiumoxid, Titandioxid und Zirkondioxid.

Diese können zwischen Zulauf 1 und Seitenabzug 4 im Abtriebsteil der ersten Kolonne angeordnet sein.

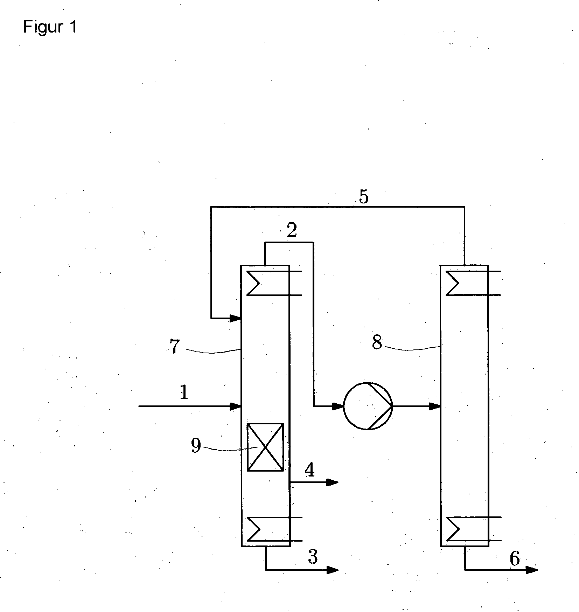

Figur 1 gibt eine Variante des erfindungsgemäßen Verfahrens wieder.

In die erste Rektifikationskolonne 7, welche beispielsweise 20 theoretische Böden umfasst, wird beispielsweise auf Höhe des 16-ten theoretischen Bodens von unten ein Einsatzstrom 1, der beispielsweise 61 Gew.-% Ethanol, 22 Gew.-% Ethylal, 15 Gew.-% Wasser und 2 Gew.-% Formaldehyd enthält, eingespeist. Die Kolonne 7 wird bei Umgebungsdruck betrieben. Die Sumpftemperatur beträgt beispielsweise 99 °C, die Kopftemperatur beträgt beispielsweise 74 °C. Die Kolonne umfasst eine Reaktionszone 9 enthaltend einen Katalysator aus saurem lonentauscherharz Diese Reaktionszone ist beispielsweise zwischen dem 11-ten und 14-ten theoretischen Boden angeordnet. Als Sumpfabzugsstrom 3 der ersten Destillationskolonne wird ein Strom aus > 98 Gew.-% Wasser erhalten. Als Seitenabzugsstrom 4 wird beispielsweise auf der Höhe des 9-ten theoretischen Boden ein Gemisch aus beispielsweise 94 Gew.-% Ethanol und 6 Gew.-% Wasser gewonnen.

Der Kopfabzugsstrom 2 der ersten Destillationskolonne enthält beispielsweise 49 Gew.-% Ethanol, 47 Gew.-% Ethylal und 4 Gew.-% Wasser. Dieser wird in die zweite Rektifikationskolonne 8, welche beispielsweise 25 theoretische Böden umfasst, beispielsweise auf der Höhe des 22-ten theoretischen Bodens eingespeist. Die Kolonne 8 wird beispielsweise bei 5 bar betrieben, die Sumpftemperatur beträgt beispielsweise 148 °C, die Kopftemperatur beträgt beispielsweise 124 °C. Der Kopfabzugsstrom 5 der zweiten Destillationskolonne 8 enthält beispielsweise 56 Gew.-% Ethanol, 40 Gew.-% Ethylal und 4 Gew.-% Wasser und wird beispielsweise auf der Höhe des 18-ten theoretischen Bodens in die erste Rektifikationskolonne 7 zurückgeführt. Der Sumpfabzugsstrom 6 der zweiten Kolonne 8 enthält beispielsweise > 99,9 Gew.-% Ethylal.

Zur Herstellung von Ethylal wird vorzugsweise Ethanol eingesetzt, das aus nachwachsenden Rohstoffen gewonnen wurde. Dieses Ethanol - auch als Bioethanol bezeichnet - weist ein charakteristisches C14 / C12-Isotopenverhältnis auf. Ethanol, welches auf Basis fossiler Rohstoffe hergestellt wurde, weist im Allgemeinen keinen Gehalt Kohlenstoff-14-Isotop auf.

Beispielsweise kann Ethanol aus folgenden nachwachsenden Rohstoffen hergestellt werden: stärke- und zuckerhaltige Pflanzen, wie z.B. Mais oder Zuckerrohr, oder cellulosehaltige Pflanzenbestandteile, z.B. Stroh, Holz, Schilf.

Zur Herstellung von Ethylal wird vorzugsweise ein Formaldehyd eingesetzt, das aus Methanol hergestellt wurde, das aus nachwachsenden Rohstoffen gewonnen wurde. Dieses Methanol weist ein charakteristisches C14/C12-Isotopenverhältnis auf, welches sich im Formaldehyd wiederfindet. Methanol, welches auf Basis fossiler Rohstoffe hergestellt wurde, weist im Allgemeinen keinen Gehalt an Kohlenstoff-14-Isotop auf.

Beispielsweise kann Methanol durch Vergasung aus folgenden nachwachsenden Rohstoffen hergestellt werden: Holz, Torf, Schilf.

Die Erfindung wird durch die nachstehenden Beispiele näher erläutert.

### Beispiele

### Beispiel 1

Der ersten Rektifikationskolonne, die bei Umgebungsdruck betrieben wird, werden 1000 kg/h eines Gemisches bestehend aus 80 Gew.-% Ethanol und 20 Gew.-% Ethylal als Einspeisungsstrom 1 zugeführt. Als Destillatstrom 2 werden 580 kg/h eines Stroms, der sich aus 56 Gew.-% Ethanol und 44 Gew.-% Ethylal zusammensetzt, am Kopf der Kolonne entnommen Dieses Gemisch wird in einer zweiten Rektifikationskolonne, die bei einem Absolutdruck von 5 bar betrieben wird, getrennt, wobei als Sumpfabzugsstrom 6 200 kg/h Ethylal mit einer Reinheit von >99,9 Gew.-% Ethylal erhalten werden. Als Kopfabzugsstrom 5 wird ein Gemisch bestehend aus 34 Gew.-% Ethanol und 66 Gew.-% Ethylal mit einem Massenstrom von 390 kg/h abgezogen. Dieses wird in die erste Rektifikationskolonne auf die oberste Stufe rückgeführt. Als weiteres Produkt wird am Sumpf der ersten Kolonne Ethanol mit einer Reinheit von 99,9 Gew.-% sowie Spuren an Ethylal als Strom 3 abgezogen.

### Beispiel 2

Der ersten Rektifikationskolonne werden 1000 kg/h eines Stroms 1 bestehend aus 60 Gew.-% Ethanol, 35 Gew.-% Wasser und 5 Gew.-% Ethylal zugeführt. Die Kolonne wird bei Umgebungsdruck betrieben. Als Kopfabzugsstrom 2 wird ein Gemisch, das sich aus 42 Gew.-% Ethanol, 4 Gew.-% Wasser und 54 Gew.-% Ethylal zusammensetzt, mit einem Massenstrom von 350 kg/h gewonnen. Am Sumpf fällt ein Gemisch aus 63 Gew.-% Ethanol und 37 Gew.-% Wasser sowie Spuren von Ethylal als Strom 3 an. Der Destillatstrom 2 wird wie in Beispiel 1 beschrieben in einer zweiten Kolonne, die bei 5 bar betrieben wird, getrennt. Als Sumpfabzugsstrom 6 fällt mit einem Massenstrom von 50 kg/h mit einer Reinheit von >99,9 Gew.-% Ethylal an. Als Kopfabzugsstrom 5 wird ein Strom bestehend aus 48 Gew.-% Ethanol, 5 Gew.-% Wasser und 47 Gew.-% Ethylal, der wieder in die erste Kolonne zurückgeführt wird, erhalten.

### Beispiel 3

Der ersten Rektifikationskolonne werden 1000 kg/h eines Stroms 1 bestehend aus 60 Gew.-% Ethanol, 35 Gew.-% Wasser und 5 Gew.-% Ethylal zugeführt. Die Kolonne wird bei Umgebungsdruck betrieben. Als Kopfabzugsstrom 2 wird ein Gemisch, das sich aus 49 Gew.-% Ethanol, 4 Gew.-% Wasser und 47 Gew.-% Ethylal zusammensetzt, mit einem Massenstrom von 400 kg/h gewonnen. Über einen Seitenabzug 4 der ersten Rektifizierkolonne, der sich zwischen Zulauf des Stroms 1 und Sumpfabzug 3 befindet, werden 710 kg/h eines Gemischs aus 85 Gew.-% Ethanol und 15 Gew.-% Wasser abgezogen. Am Sumpf der ersten Rektifikationskolonne fallen 240 kg/h Wasser mit einer Reinheit >99,9 Gew.-% als Strom 3 an. Der Strom 2 wird wie in Beispiel 1 beschrieben in einer zweiten Kolonne, die bei 5 bar betrieben wird, aufgetrennt. Das Sumpfprodukt fällt mit einem Massenstrom von 50 kg/h als Strom 6 an und besteht zu >99,9 Gew.-% aus Ethylal. Als *Kopfabzugsstrom* 5 wird ein Strom bestehend aus 56 Gew.-% Ethanol, 4 Gew.-% Wasser und 40 Gew.-% Ethylal erhalten, der wieder in die erste Kolonne zurückgeführt wird.

### Beispiel 4

Der ersten Rektifikationskolonne werden 1000 kg/h eines Stroms 1 bestehend aus 61 Gew.-% Ethanol, 16,5 Gew.-% Wasser, 22 Gew.-% Ethylal und 0,5 Gew-% Formaldehyd zugeführt. Die Kolonne wird bei Umgebungsdruck betrieben. Als Kopfabzugsstrom 2 wird ein Gemisch, das sich aus 49 Gew.-% Ethanol, 4 Gew.-% Wasser und 47 Gew.-% Ethylal zusammensetzt, mit einem Massenstrom von 1886 kg/h gewonnen. Über einen Seitenabzug 4 der ersten Rektifizierkolonne, der sich zwischen Zulauf des Stroms 1 und Sumpfabzug 3 befindet, werden 650 kg/h eines Gemischs aus 94 Gew.-% Ethanol und 6 Gew.-% Wasser abgezogen. Am Sumpf der ersten Rektifikationskolonne fallen 131 kg/h Wasser mit einer Reinheit >96 Gew.-% als Strom 3 an. Der Strom enthält neben Wasser auch 3.8 Gew.-% Formaldehyd. Der Strom 2 wird wie in Beispiel 1 beschrieben in einer zweiten Kolonne, die bei 5 bar betrieben wird, aufgetrennt. Das Sumpfprodukt fällt mit einem Massenstrom von 220 kg/h als Strom 6 an und besteht zu >99,9 Gew.-% aus Ethylal. Als Kopfabzugsstrom 5 wird ein Strom bestehend aus 56 Gew.-% Ethanol, 4 Gew.-% Wasser und 40 Gew.-% Ethylal erhalten, der wieder in die erste Kolonne zurückgeführt wird.

### Beispiel 5

In einer Rektifikationskolonne 7, welche 20 theoretische Böden umfasst, wird auf Höhe des 16-ten theoretischen Bodens (von unten gezählt) ein Einsatzstrom 1 von 1000 kg/h, der 61 Gew.-% Ethanol, 22 Gew.-% Ethylal, 15 Gew.-% Wasser und 2 Gew.-% Formaldehyd enthält, eingespeist. Die Kolonne 7 wird bei Umgebungsdruck betrieben. Die Sumpftemperatur beträgt 99 °C, die Kopftemperatur beträgt 74 °C. Die Kolonne umfasst eine Reaktionszone enthaltend einen Katalysator aus saurem lonentauscherharz. Diese Reaktionszone ist zwischen dem 11-ten und 14-ten theoretischen Boden angeordnet. Als Sumpfabzugsstrom 3 der ersten Destillationskolonne wird ein Strom von 127 kg/h bestehend aus 98 Gew.-% Wasser und 2 Gew.% Formaldehyd erhalten. Als Seitenabzugsstrom 4 werden auf der Höhe des 9-ten theoretischen Bodens 591 kg/h eines Gemisches aus 94 Gew.-% Ethanol und 6 Gew.-% Wasser gewonnen.

Der Kopfabzugsstrom 2 der ersten Destillationskolonne von 2421 kg/h enthält 49 Gew.-% Ethanol, 47 Gew.-% Ethylal und 4 Gew.-% Wasser. Dieser wird in die zweite Rektifikationskolonne 8, welche 25 theoretische Böden umfasst, auf der Höhe des 22-ten theoretischen Bodens eingespeist. Die Kolonne 8 wird bei 5 bar betrieben, die Sumpftemperatur beträgt 148 °C, die Kopftemperatur beträgt 124 °C. Der Kopfabzugsstrom 5 der zweiten Destillationskolonne 8 von 2138 kg/h enthält 56 Gew.-% Ethanol, 40 Gew.-% Ethylal und 4 Gew.-% Wasser und wird auf der Höhe des 18-ten theoretischen Bodens in die erste Rektifikationskolonne 7 zurückgeführt. Der Sumpfabzugsstrom 6 der zweiten Kolonne 8 von 282 kg/h enthält 99,9 Gew.-% Ethylal.

## Patentansprüche

1. Verfahren zur Abtrennung von Ethylal aus einem Einsatzstrom 1, der Ethanol, Ethylal und gegebenenfalls Wasser enthält, bei dem
a) ein Einsatzstrom 1, der Ethanol und Ethylal enthält, bereitgestellt wird,
b) der Einsatzstrom 1 und ein Rückführstrom 5, der Ethanol und Ethylal enthält, In eine erste Destillationsstufe eingespeist und bei einem Druck von 0,1 bis 32 bar destilliert werden, wobei ein Strom 2, der Ethanol und Ethylal enthält, wobei dieser Strom Ethylal in höherer Konzentration als Strom 1 enthält, erhalten wird,
c) der Strom 2 in einer zweiten Destillationsstufe bei einem Druck von 1,1 bis 33 bar destilliert wird, wobei der Druck in der zweiten Destillationsstufe um 1,0 bis 10 bar höher als der Druck in der ersten Destillationsstufe ist, wobei ein Rückführstrom 5, der Ethanol und Ethylal enthält, und ein Strom 6, der im Wesentlichen aus Ethylal besteht, erhalten werden.

2. Verfahren nach Anspruch 1, **dadurch gekennzeichnet, dass** die erste Destillationsstufe bei einem Druck von 0,25 bis 1,5 bar durchgeführt wird.

3. Verfahren nach Anspruch 1 oder 2, **dadurch gekennzeichnet, dass** die erste Destillationsstufe in einer ersten Destillationskolonne mit mindestens 2 theoretischen Böden und die zweite Destillationsstufe in einer zweiten Destillationskolonne mit mindestens 2 theoretischen Böden durchgeführt werden.

4. Verfahren nach Anspruch 3, **dadurch gekennzeichnet, dass** der Strom 2 als Kopfabzugsstrom und der Strom 6 als Sumpfabzugsstrom erhalten werden.

5. Verfahren nach Anspruch 3 oder 4, **dadurch gekennzeichnet, dass** der Einsatzstrom 1 im Wesentlichen aus Ethanol und Ethylal besteht, und in Schritt b) ein Strom 3, der im Wesentlichen aus Ethanol besteht, als Sumpfabzugsstrom erhalten wird.

6. Verfahren nach einem der Ansprüche 3 oder 4, **dadurch gekennzeichnet, dass** der Einsatzstrom 1 Wasser enthält und in Schritt b) ein Strom 3, der im Wesentlichen aus Ethanol und Wasser besteht, als Sumpfabzugsstrom erhalten wird.

7. Verfahren nach einem der Ansprüche 3 oder 4, **dadurch gekennzeichnet, dass** der Einsatzstrom 1 Wasser enthält, und in Schritt b) ein Strom 4, der Ethanol und Wasser enthält, als Seitenabzugsstrom und ein Strom 3, der im Wesentlichen aus Wasser besteht, als Sumpfabzugsstrom erhalten werden.

8. Verfahren nach einem der Ansprüche 3 bis 7, **dadurch gekennzeichnet, dass** der Einsatzstrom 1 Leichtsieder enthält, und in Schritt c) der Rückführstrom 5 als Seitenabzugsstrom und ein Strom 7, der Leichtsieder enthält, als Kopfabzugsstrom erhalten werden.

9. Verfahren nach Anspruch 3 oder 4, **dadurch gekennzeichnet, dass** der Einsatzstrom 1 Formaldehyd enthält.

10. Verfahren nach Anspruch 9, **dadurch gekennzeichnet, dass** der Einsatzstrom 1 Formaldehyd und Wasser enthält, und in Schritt b) ein Strom 3, der im Wesentlichten aus Ethanol, Wasser und Formaldehyd besteht, als Sumpfabzugsstrom erhalten wird.

11. Verfahren nach Anspruch 9, **dadurch gekennzeichnet, dass** der Einsatzstrom 1 Formaldehyd und Wasser enthält, und In Schritt b) ein Strom 4, der Ethanol und Wasser enthält, als Seitenabzugsstrorn und ein Strom 3, der im Wesentlichen aus Wasser und Formaldehyd besteht, als Sumpfabzugsstrom erhalten werden.

12. Verfahren nach einem der Ansprüche 9 bis 11, **dadurch gekennzeichnet, dass** im Abtriebsteil der ersten Kolonne eine Reaktionszone umfassend einen sauren heterogenen Katalysator angeordnet ist, in der Formaldehyd und Ethanol zu Ethylal reagieren.

13. Verfahren nach einem der Ansprüche 1 bis 12, **dadurch gekennzeichnet, dass** der Einsatzstrom 1 ein Reaktionsgemisch enthält, welches bei der sauer katalysierten Synthese von Ethylal aus Formaldehyd und Ethanol anfällt.

14. Verfahren nach Anspruch 13, **dadurch gekennzeichnet, dass** bei der Synthese von Ethylal Ethanol, welches aus nachwachsenden Rohstoffen erhalten wurde, eingesetzt wird.

## Claims

1. Process for removing ethylal from a feed steam 1 comprising ethanol and ethylal, with or without water, in which
a) a feed steam 1 comprising ethanol and ethylal is provided,
b) feed steam 1 and a recycle stream 5 comprising ethanol and ethylal are fed into a first distillation stage and distilled at a pressure of 0.1 to 32 bar to obtain a stream 2 comprising ethanol and ethylal, said stream comprising ethylal in a higher concentration than stream 1,
c) stream 2 is distilled in a second distillation stage at a pressure of 1.1 to 33 bar, the pressure in the second distillation stage being 1.0 to 10 bar higher than the pressure in the first distillation stage, to obtain a recycle stream 5 comprising ethanol and ethylal, and a stream 6 consisting essentially of ethylal.

2. Process according to Claim 1, **characterized in that** the first distillation stage is performed at a pressure of 0.25 to 1.5 bar.

3. Process according to Claim 1 or 2, **characterized in that** the first distillation stage is performed in a first distillation column with at least 2 theoretical plates, and the second distillation stage in a second distillation column with at least 2 theoretical plates.

4. Process according to Claim 3, **characterized in that** stream 2 is obtained as a top draw stream and stream 6 as a bottom draw stream.

5. Process according to Claim 3 or 4, **characterized in that** feed steam 1 consists essentially of ethanol and ethylal, and a stream 3 consisting essentially of ethanol is obtained as a bottom draw stream in step b).

6. Process according to either of Claims 3 and 4, **characterized in that** feed steam 1 comprises water and, in step b), a stream 3 consisting essentially of ethanol and water is obtained as a bottom draw stream.

7. Process according to either of Claims 3 and 4, **characterized in that** feed steam 1 comprises water and, in step b), a stream 4 comprising ethanol and water is obtained as a side draw stream, and a stream 3 consisting essentially of water as a bottom draw stream.

8. Process according to any of Claims 3 to 7, **characterized in that** feed steam 1 comprises low boilers and, in step c), recycle stream 5 is obtained as a side draw stream, and a stream 7 comprising low boilers as a top draw stream.

9. Process according to Claim 3 or 4, **characterized in that** feed steam 1 comprises formaldehyde.

10. Process according to Claim 9, **characterized in that** feed steam 1 comprises formaldehyde and water, and, in step b), a stream 3 consisting essentially of ethanol, water and formaldehyde is obtained as a bottom draw stream.

11. Process according to Claim 9, **characterized in that** feed steam 1 comprises formaldehyde and water, and, in step b), a stream 4 comprising ethanol and water is obtained as a side draw stream, and a stream 3 consisting essentially of water and formaldehyde as a bottom draw stream.

12. Process according to any of Claims 9 to 11, **characterized in that** a reaction zone comprising an acidic heterogeneous catalyst is disposed in the stripping section of the first column, and formaldehyde and ethanol react therein to give ethylal.

13. Process according to any of Claims 1 to 12, **characterized in that** feed steam 1 comprises a reaction mixture obtained in the acid-catalyzed synthesis of ethylal from formaldehyde and ethanol.

14. Process according to Claim 13, **characterized in that** ethanol which has been obtained from renewable raw materials is used in the synthesis of ethylal.

## Revendications

1. Procédé de séparation d'éthylal d'un courant d'alimentation 1, qui contient de l'éthanol, de l'éthylal et éventuellement de l'eau, selon lequel
a) un courant d'alimentation 1, qui contient de l'éthanol et de l'éthylal, est mis à disposition,
b) le courant d'alimentation 1 et un courant de recyclage 5, qui contient de l'éthanol et de l'éthylal, sont introduits dans une première étape de distillation et distillés à une pression de 0,1 à 32 bar, un courant 2, qui contient de l'éthanol et de l'éthylal, ce courant contenant de l'éthylal en une concentration plus élevée que le courant 1, étant obtenu,
c) le courant 2 est distillé dans une deuxième étape de distillation à une pression de 1,1 à 33 bar, la pression dans la deuxième étape de distillation étant 1,0 à 10 bar supérieure à la pression dans la première étape de distillation, un courant de recyclage 5, qui contient de l'éthanol et de l'éthylal, et un courant 6, qui est essentiellement constitué d'éthylal, étant obtenus.

2. Procédé selon la revendication 1, **caractérisé en ce que** la première étape de distillation est réalisée à une pression de 0,25 à 1,5 bar.

3. Procédé selon la revendication 1 ou 2, **caractérisé en ce que** la première étape de distillation est réalisée dans une première colonne de distillation contenant au moins 2 plateaux théoriques et la deuxième étape de distillation dans une deuxième colonne de distillation contenant au moins 2 plateaux théoriques.

4. Procédé selon la revendication 3, **caractérisé en ce que** le courant 2 est obtenu en tant que courant de sortie de tête et le courant 6 en tant que courant de sortie de fond.

5. Procédé selon la revendication 3 ou 4, **caractérisé en ce que** le courant d'alimentation 1 est essentiellement constitué d'éthanol et d'éthylal, et, à l'étape b), un courant 3, qui est essentiellement constitué d'éthanol, est obtenu en tant que courant de sortie de fond.

6. Procédé selon l'une quelconque des revendications 3 ou 4, **caractérisé en ce que** le courant d'alimentation 1 contient de l'eau et, à l'étape b), un courant 3, qui est essentiellement constitué d'éthanol et d'eau, est obtenu en tant que courant de sortie de fond.

7. Procédé selon l'une quelconque des revendications 3 ou 4, **caractérisé en ce que** le courant d'alimentation 1 contient de l'eau et, à l'étape b), un courant 4, qui contient de l'éthanol et de l'eau, est obtenu en tant que courant de sortie latérale, et un courant 3, qui est essentiellement constitué d'eau, est obtenu en tant que courant de sortie de fond.

8. Procédé selon l'une quelconque des revendications 3 à 7, **caractérisé en ce que** le courant d'alimentation 1 contient des composants de point d'ébullition bas, et, à l'étape c), le courant de recyclage 5 est obtenu en tant que courant de sortie latérale et un courant 7, qui contient des composants de point d'ébullition bas, est obtenu en tant que courant de sortie de tête.

9. Procédé selon la revendication 3 ou 4, **caractérisé en ce que** le courant d'alimentation 1 contient du formaldéhyde.

10. Procédé selon la revendication 9, **caractérisé en ce que** le courant d'alimentation 1 contient du formaldéhyde et de l'eau et, à l'étape b), un courant 3, qui est essentiellement constitué d'éthanol, d'eau et de formaldéhyde, est obtenu en tant que courant de sortie de fond.

11. Procédé selon la revendication 9, **caractérisé en ce que** le courant d'alimentation 1 contient du formaldéhyde et de l'eau et, à l'étape b), un courant 4, qui contient de l'éthanol et de l'eau, est obtenu en tant que courant de sortie latérale, et un courant 3, qui est essentiellement constitué d'eau et de formaldéhyde, est obtenu en tant que courant de sortie de fond.

12. Procédé selon l'une quelconque des revendications 9 à 11, **caractérisé en ce qu'**une zone de réaction comprenant un catalyseur hétérogène acide, dans laquelle le formaldéhyde et l'éthanol sont transformés en éthylal, est agencée dans la zone de rectification de la première colonne.

13. Procédé selon l'une quelconque des revendications 1 à 12, **caractérisé en ce que** le courant d'alimentation 1 contient un mélange réactionnel qui se forme lors de la synthèse sous catalyse acide d'éthylal à partir de formaldéhyde et d'éthanol.

14. Procédé selon la revendication 13, **caractérisé en ce que** de l'éthanol obtenu à partir de matières premières renouvelables est utilisé lors de la synthèse d'éthylal.
